# EUROPEAN PATENT APPLICATION

(11) **EP 4 502 776 A1**
(43) Date of publication of application: **05.02.2025**
(21) Application number: 23779576.0
(22) Date of filing: 14.03.2023
(51) Int. Cl.: G06F 3/04845, A61B 5/11, G06F 3/01, G06F 3/0346, G06F 3/038, G06F 3/04815, G06T 19/00

(54) **INFORMATION PROCESSING SYSTEM AND INFORMATION PROCESSING METHOD**

(30) Priority: 31.03.2022 JP 2022060150
(71) Applicant: Sony Group Corporation, Tokyo 108-0075 (JP)
(72) Inventor: YAMAGISHI Kazuko, Tokyo 108-0075 (JP); OMATA Takanobu, Tokyo 108-0075 (JP); SHIMIZU Itaru, Tokyo 108-0075 (JP); SAITO Mari, Tokyo 108-0075 (JP); KURIHARA Takayuki, Tokyo 108-0075 (JP)
(74) Representative: D Young & Co LLP
(86) International application number: PCT/JP2023/009905
(87) International publication number: WO 2023/189558

(57) **Abstract**

The present technology relates to an information processing system and an information processing method capable of suitably reducing an immersive feeling of a user.

An information control system of the present technology includes a presentation control unit that presents content including an object to a user and changes the object according to a stage of interaction of the user with the object at a timing according to an immersion degree of the user with respect to the content. The presentation control unit changes a plurality of types of visual characteristics of the object. Furthermore, the presentation control unit changes a visual characteristic including at least one of a position, a depth, a motion, a size, or a shape of the object. The present technology can be applied to a system that provides VR or AR experience.

## Description

### TECHNICAL FIELD

The present technology relates to an information processing system and an information processing method, and more particularly, to an information processing system and an information processing method capable of suitably reducing an immersive feeling of a user.

### BACKGROUND ART

There is a technology for allowing a user to experience virtual reality (VR) or augmented reality (AR) by presenting an image corresponding to a field of view on a virtual space or superimposing and presenting a virtual object in reality.

Users experiencing VR may experience so-called VR sickness. As a technique for reducing VR sickness, for example, Patent Document 1 describes a technique for reducing visibility of an object. Furthermore, as a technique for reducing VR sickness, Patent Document 2 describes a technique for performing processing of reducing an immersive feeling on video data.

### CITATION LIST

### PATENT DOCUMENT

Patent Document 1: Japanese Patent Application Laid-Open No. 2019-75091
Patent Document 2: Japanese Patent Application Laid-Open No. 2021-180425

### SUMMARY OF THE INVENTION

### PROBLEMS TO BE SOLVED BY THE INVENTION

As the development of the VR technology and the AR technology advances, the reality of the virtual object and the virtual space increases, and the boundary between the reality and the virtual reality becomes unclear, so that it is conceivable that the experience of VR and AR adversely affects the user's mind and body. In order to reduce the influence on the user's mind and body, it is necessary to reduce excessive immersive feeling and realistic feeling.

However, in the conventional technique, the visual presentation is controlled from the beginning, which may make it difficult for the user to obtain an immersive feeling. Furthermore, in the conventional technology, since the control of the visual presentation is not in accordance with the cognitive characteristics of the person, there is a possibility that it is difficult for the user to obtain an immersive feeling or a psychological burden is applied to the user due to the rapid control of the visual presentation.

The present technology has been made in view of such a situation, and an object thereof is to suitably reduce an immersive feeling of a user.

### SOLUTIONS TO PROBLEMS

An information control system according to one aspect of the present technology includes a presentation control unit that presents content including an object to a user and changes the object according to a stage of interaction of the user with the object at a timing according to an immersion degree of the user with respect to the content.

In an information control method according to one aspect of the present technology, an information processing system is configured to: present content including an object to a user; and change the object according to a stage of interaction of the user with the object at a timing according to an immersion degree of the user with respect to the content.

In one aspect of the present technology, content including an object is presented to a user, and the object is changed according to a stage of interaction of the user with the object at a timing according to an immersion degree of the user with respect to the content.

### BRIEF DESCRIPTION OF DRAWINGS

Fig. 1 is a block diagram illustrating a configuration example of an information processing system according to an embodiment of the present technology.
Fig. 2 is a diagram illustrating an example of an interaction detection method.
Fig. 3 is a diagram illustrating an example of a scale of an immersion degree.
Fig. 4 is a diagram illustrating a path in the brain of a person who processes visual information.
Fig. 5 is a diagram illustrating an example of information necessary in each stage of a grasping movement.
Fig. 6 is a diagram illustrating an example of a method of detecting each stage of the grasping movement in the information processing system and an intervention method according to each stage.
Fig. 7 is a flowchart for explaining processing performed by the information processing system in a case where immersion is not desired from the beginning.
Fig. 8 is a flowchart for explaining processing performed by the information processing system in a case where it is desired to maintain an appropriate immersive state.
Fig. 9 is a flowchart for explaining processing performed by the information processing system 1 in a case where it is desired to return from an immersive state to reality.
Fig. 10 is a diagram illustrating use cases of the information processing system.
Fig. 11 is a diagram illustrating an example of problems in each use case and an intervention method of the information processing system.
Fig. 12 is a diagram illustrating an example of a scene of a use case.
Fig. 13 is a diagram illustrating another example of a scene of a use case.
Fig. 14 is a block diagram illustrating a configuration example of hardware of a computer.

### MODE FOR CARRYING OUT THE INVENTION

Hereinafter, modes for carrying out the present technology will be described. The description will be given in the following order.
1. Configuration of Information Processing System
2. Operation of Information Processing System
3. Use Case
4. Modifications

### <1. Configuration of Information Processing System>

There is a technology for allowing a user to experience virtual reality (VR) or augmented reality (AR) by presenting an image corresponding to a field of view on a virtual space or superimposing and presenting a virtual object in reality.

As the development of the VR technology and the AR technology advances, the reality of the virtual object and the virtual space increases, and the boundary between the reality and the virtual reality becomes unclear, so that it is conceivable that the experience of VR and AR adversely affects the user's mind and body. In order to reduce the influence on the user's mind and body, it is necessary to reduce excessive immersive feeling and realistic feeling.

However, in the conventional technique, the visual presentation is controlled from the beginning, which may make it difficult for the user to obtain an immersive feeling. Furthermore, in the conventional technology, since the control of the visual presentation is not in accordance with the cognitive characteristics of the person, there is a possibility that it is difficult for the user to obtain an immersive feeling or a psychological burden is applied to the user due to the rapid control of the visual presentation.

It is preferable to return from the immersive state to the non-immersive state by unconsciously working on the user so as not to apply a psychological burden as much as possible, but the mechanism of the immersive feeling in the virtual space has not been clarified, and there are few effective means.

An embodiment of the present technology has been conceived by focusing on the above points, and proposes a technology capable of reducing the "authenticity" of a virtual object in consideration of cognitive characteristics of a person without applying a psychological burden, and giving a feeling of strangeness to a user, thereby preventing excessive immersion in a virtual space. Hereinafter, the present embodiment will be described in detail.

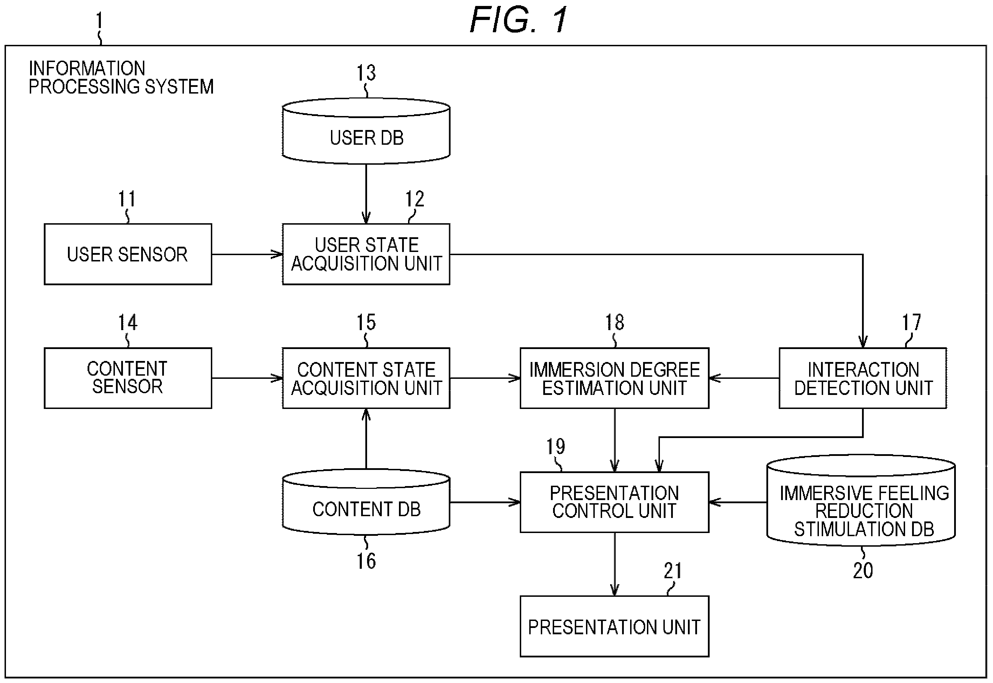

Fig. 1 is a block diagram illustrating a configuration example of an information processing system 1 according to an embodiment of the present technology.

An information processing system 1 in Fig. 1 is, for example, a system that presents content including a virtual object to a user and allows the user to experience VR and AR.

As illustrated in Fig. 1, the information processing system 1 includes a user sensor 11, a user state acquisition unit 12, a user database (DB) 13, a content sensor 14, a content state acquisition unit 15, a content DB 16, an interaction detection unit 17, an immersion degree estimation unit 18, a presentation control unit 19, an immersive feeling reduction stimulation DB 20, and a presentation unit 21.

The user sensor 11 includes a biometric sensor, a camera, a microphone, and the like. The user sensor 11 detects biological information, facial expression, voice, and the like of the user, and supplies sensor data as a detection result to the user state acquisition unit 12.

The user state acquisition unit 12 acquires the state of the user on the basis of the sensor data supplied from the user sensor 11. In addition, the user state acquisition unit 12 acquires, from the user DB 13, user information indicating an attribute of the user, information of the user related to vision, a play history of a past game of the user, and the like. The attribute of the user includes the age group and gender of the user, and the information of the user related to vision includes the eyesight of the user.

The user state acquisition unit 12 supplies information indicating the state of the user and the user information to the interaction detection unit 17.

In the user DB 13, user information is registered in advance.

The content sensor 14 includes a camera, a microphone, and the like. The content sensor 14 detects video, audio, and the like of content presented to the user, and supplies sensor data as a detection result to the content state acquisition unit 15. Note that the content sensor 14 can also acquire the object ID of the virtual object presented to the user as sensor data.

The content state acquisition unit 15 acquires the scene and the viewing state of the content on the basis of the sensor data supplied from the content sensor. The viewing state of the content includes a device used by the user, a reproduction volume of audio of the content, and a reproduction speed of the content.

In addition, the content state acquisition unit 15 acquires content information including the immersive feeling rate of the content and the intervention type information from the content DB 16.

The immersive feeling rate is information regarding ease of immersion, such as authenticity of a virtual object included in content and ease of drawing attention to the virtual object. The intervention type information is information indicating a target value of the immersion degree (a degree to which immersion is not desired), a timing of presenting a stimulus for reducing the immersion degree, a method of presenting a stimulus, and the like. The immersive feeling rate and the intervention type information are set for each scene of the content. The content state acquisition unit 15 acquires content information corresponding to a scene presented to the user.

The content state acquisition unit 15 supplies information indicating the viewing state of the content and content information corresponding to the scene of the content to the immersion degree estimation unit 18.

Content information is registered in the content DB 16 in advance.

The interaction detection unit 17 detects the interaction of the user with the virtual object on the basis of the information supplied from the user state acquisition unit 12.

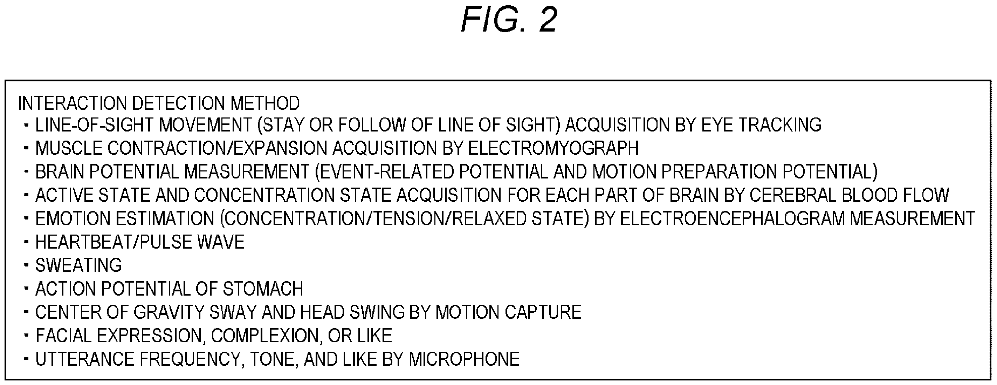

Fig. 2 is a diagram illustrating an example of an interaction detection method.

As illustrated in Fig. 2, the interaction detection unit 17 acquires a line-of-sight movement of the user such as stay or follow of the line-of-sight with respect to the virtual object by, for example, eye tracking, and detects an interaction of the user with respect to the virtual object on the basis of the line-of-sight movement of the user. Furthermore, the interaction detection unit 17 acquires contraction and extension of the user's muscle by, for example, an electromyograph, and detects the user's interaction with the virtual object on the basis of the expansion and contraction of the muscle.

The interaction detection unit 17 acquires, for example, an event-related potential or a motion preparation potential by brain potential measurement, and detects the user's interaction with the virtual object on the basis of these brain potentials. The interaction detection unit 17 acquires, for example, an active state and a concentration state for each part of the brain of the user on the basis of the cerebral blood flow, and detects the interaction of the user with the virtual object on the basis of the state for each part of the brain.

The interaction detection unit 17 acquires a result of user's emotion estimation based on electroencephalogram measurement, such as a concentration state, a tension state, or a relaxed state, for example, and detects the user's interaction with the virtual object on the basis of the emotion estimation result. The interaction detection unit 17 detects the interaction of the user with the virtual object on the basis of, for example, a heartbeat, a pulse wave, sweating, or an action potential of the stomach of the user.

The interaction detection unit 17 acquires, for example, a center of gravity sway and a head swing of the user by motion capture, and detects the interaction of the user with the virtual object on the basis of these swings. The interaction detection unit 17 detects the user's interaction with the virtual object on the basis of, for example, the user's facial expression, complexion, or the like. The interaction detection unit 17 acquires the frequency, tone, and the like of the user's utterance by the microphone, for example, and detects the user's interaction with the virtual object on the basis of the information regarding the voice.

Note that the user's line-of-sight movement, muscle expansion and contraction, brain potential, a state of each part of the brain, a result of emotion estimation, a heartbeat, a pulse wave, sweating, a stomach action potential, a swing of the center of gravity or the head, a facial expression, a complexion, information regarding voice, and the like used for detection of interaction are information acquired by the user state acquisition unit 12 as information indicating the user's state.

Returning to Fig. 1, the interaction detection unit 17 supplies a detection result of the interaction to the immersion degree estimation unit 18 and the presentation control unit 19.

The immersion degree estimation unit 18 estimates the immersion degree of the user in the content on the basis of the information supplied from the content state acquisition unit 15 and the interaction detection unit 17. Specifically, the immersion degree estimation unit 18 estimates the immersive feeling obtained by the user for the content on the basis of the content of the interaction of the user with the virtual object, and rates the immersion degree. In a case where content in which the user interacts with the information processing system 1 is presented, the immersion degree estimation unit 18 can also estimate the immersion degree on the basis of the user's reaction to the interactive interaction between the user and the avatar embodying the information processing system 1.

Note that the immersion degree is scaled in advance on the basis of subjective evaluation using an evaluation scale relating to the immersive feeling, such as the realistic feeling, the sense of operation, and the sense of physical possession.

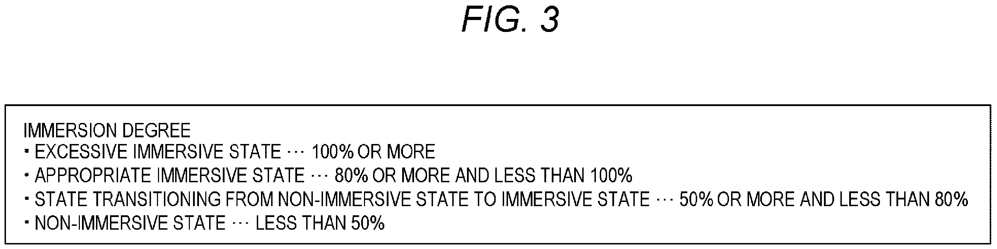

Fig. 3 is a diagram illustrating an example of a scale of an immersion degree.

In the example of Fig. 3, an excessive immersive state is indicated with an immersion degree of 100% or more, and an appropriate immersive state is indicated with an immersion degree of 80% or more and less than 100%. Furthermore, a state transitioning from a non-immersive state to an immersive state is indicated with an immersion degree of 50% or more and less than 80%, and a non-immersive state is indicated with an immersion degree of less than 50%. Note that the value of the immersion degree illustrated in Fig. 3 is an example, and the immersion degree may be a non-quantitative value such as a relative evaluation value or a feature amount defined by machine learning.

Returning to Fig. 1, the immersion degree estimation unit 18 supplies the estimated immersion degree to the presentation control unit 19.

The presentation control unit 19 controls the presentation unit 21 to present the content to the user. Furthermore, the presentation control unit 19 controls the presentation unit 21 to intervene in the interaction in consideration of the cognitive characteristics of the person, thereby reducing the immersion degree of the user.

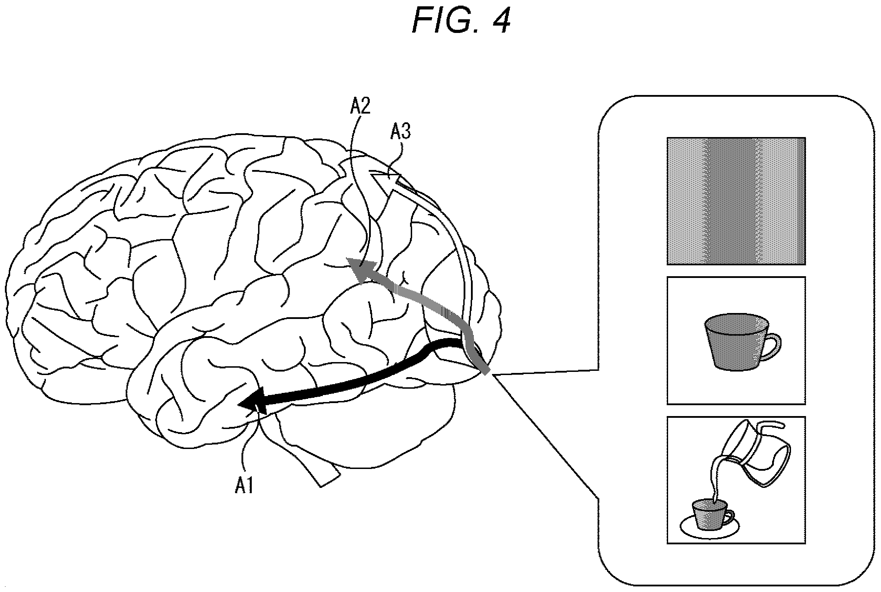

Fig. 4 is a diagram illustrating a path in the brain of a person who processes visual information.

The visual information obtained visually is processed in the ventral path, the ventrodorsal path, and the dorsodorsal path indicated by arrows A1 to A3 in Fig. 4 in the human brain. The ventral path has a function related to analyzing the position and movement of the motion target and being conscious of the motion target. The dorsal path including the ventrodorsal path and the dorsodorsal path has a function of processing information of the position, movement, and shape of the motion target in a less conscious manner and causing an appropriate motion.

First, in the human brain, the visual information that has reached the starting point of the arrow illustrated in Fig. 4 is sequentially sent forward along each path, and the processing proceeds. In the ventral path and the ventrodorsal path, as illustrated in the upper part of the speech balloon, specific position information of the visual field such as whether a part of the visual field is gray or moving is processed. Next, as illustrated in the middle part of the speech balloon, the human brain processes not only a specific visual field but also visual characteristics such as a cylindrical shape or how many objects (cups) the subject looks, with the ventral path and the ventrodorsal path. Next, as illustrated in the lower part of the speech balloon, the human brain has knowledge about the subject and determines that the subject is present, such as using the subject to put a beverage in a cylindrical shape. Here, the determination is made using not only visual information but also information beyond the type of sensation. In the dorsodorsal path, such a change is not clear probably because the processing result does not need to be conscious.

The presentation control unit 19 intervenes in the interaction in a non-conscious manner by controlling the visual characteristics of the virtual object processed using the dorsal path in the user's brain and working only unconsciously. By intervening in the interaction in an unconscious manner, the information processing system 1 can reduce the immersion degree in the content without applying a psychological burden to the user.

Here, the control of the visual characteristics of the virtual object is performed according to the stage of the interaction detected by the interaction detection unit 17.

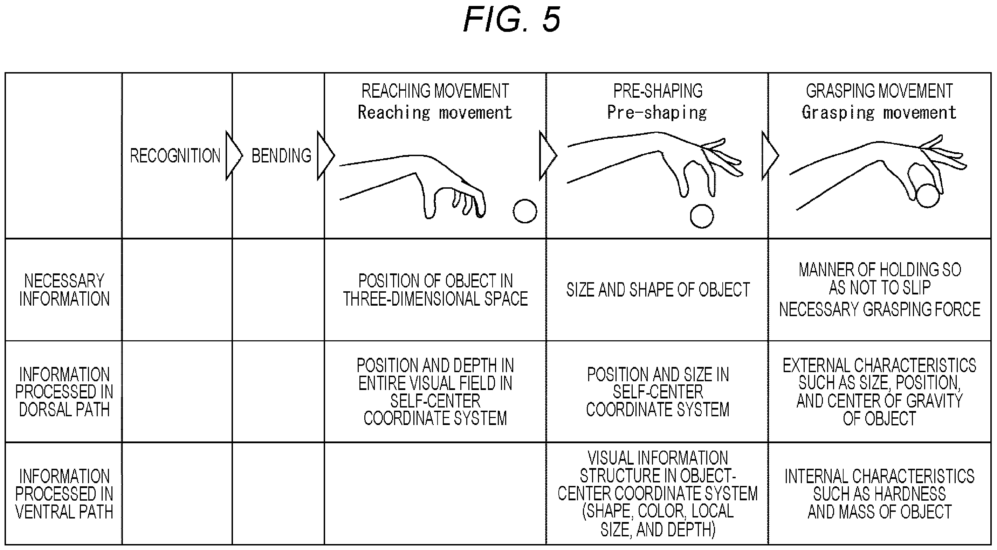

Fig. 5 is a diagram illustrating an example of information necessary in each stage of the grasping movement. Hereinafter, it is assumed that a grasping movement is detected as an interaction.

As illustrated in the upper part of Fig. 5, the motion in which the person grips the object is divided into five stages of recognition, bending, reaching movement, pre-shaping, and grasping movement. The stage of recognition indicates that the person stays the line-of-sight or pays attention to the object to be grasped, and the stage of bending indicates that the person bends the elbow or changes the center of gravity. The stage of the reaching movement indicates that the person moves the hand toward the object to be grasped, and the stage of the pre-shaping indicates that the distance between the fingers is increased to grasp the object to be grasped. The stage of the grasping movement indicates that a person grasps an object to be grasped with a hand.

At the stage of the reaching movement, information on the position of the object to be grasped in the three-dimensional space is required. In the human brain, as information on the position of the object to be grasped in the three-dimensional space, information on the position and depth in the entire visual field in the self-center coordinate system is processed in the dorsal path.

In the pre-shaping stage, it is necessary to acquire information on the size and shape of the object to be grasped by object recognition. In the human brain, as information on the size and shape of the object to be grasped, information on the position and size in the self-center coordinate system is processed on the dorsal path, and visual information in the object-center coordinate system is processed on the ventral path. The visual information processed in the ventral path includes shape, color, local size, and depth information.

In the stage of the grasping movement, it is necessary to predict a manner of holding the object to be grasped so as not to slip and a grasping force necessary for grasping the object to be grasped on the basis of the visual information. In the human brain, as visual information necessary for prediction, external characteristics such as a size, a position, and a center of gravity of an object to be grasped are processed in the dorsal path, and internal characteristics such as hardness and mass of the object to be grasped are processed in the ventral path. The external characteristics of the object can be specified only by the visual information, but the internal characteristics of the object cannot be specified only by the visual information. Therefore, the external characteristics of the object are specified not only by the visual information but also by accessing knowledge. By mutually utilizing the external characteristics processed in the dorsal path and the internal characteristics processed in the ventral path, a manner of holding the object to be grasped so as not to slip and a grasping force necessary for grasping the object to be grasped are predicted.

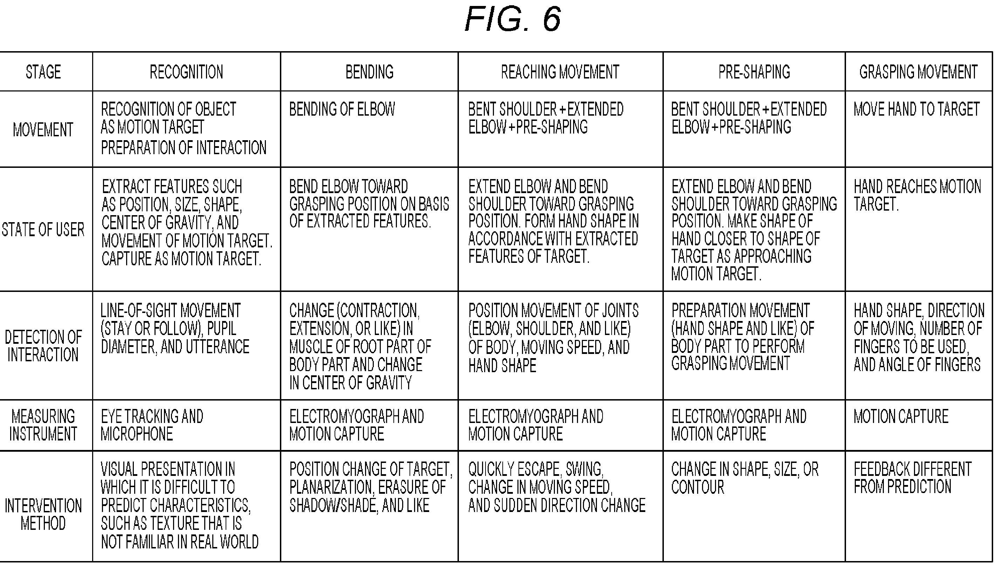

Fig. 6 is a diagram illustrating an example of a method of detecting each stage of the grasping movement in the information processing system 1 and an intervention method corresponding to each stage.

As illustrated in Fig. 6, at the stage of recognition, a virtual object as a target to be grasped is recognized, and preparation for interaction is performed. At this time, the user extracts features such as the position, size, shape, center of gravity, and movement of the virtual object as the recognition target, and captures the virtual object as the recognition target as the target to be grasped (motion target). The appearance of the cognitive movement is detected on the basis of the user's line-of-sight movement and pupil diameter acquired by the eye tracking, the user's utterance acquired by the microphone, and the like.

At the timing of the recognition stage, the presentation control unit 19 performs visual presentation in which it is difficult to predict the characteristics, such as changing the texture of the virtual object to a texture that is not familiar in reality.

In the bending stage, the elbow is bent. At this time, the user bends the elbow toward the grasping position on the basis of the extracted characteristics. The appearance of the bending movement is detected on the basis of a change in contraction, extension, or the like of the muscle of the root part of the body part of the user acquired by an electromyograph, a change in the center of gravity of the user acquired by motion capture, or the like.

At the timing of the bending stage, the presentation control unit 19 changes the position of the virtual object to be grasped, planarizes the virtual object, erases shadow/shade of the virtual object, and the like.

In the stage of the reaching movement, the shoulder is bent, the elbow is extended, and pre-shaping is performed. At this time, the user extends the elbow and bends the shoulder toward the grasping position. In addition, the user forms a hand shape in accordance with the extracted characteristics. The appearance of the reaching movement is detected on the basis of the position movement of the joints of the body such as the elbow and the shoulder of the user, the shape of the hand, and the like acquired by an electromyograph or the motion capture.

At the timing of the reaching movement stage, the presentation control unit 19 performs visual presentation in which the virtual object to be grasped quickly escapes, a change in the moving speed of the virtual object, visual presentation in which the virtual object suddenly changes its direction, and the like.

In the pre-shaping stage, the shoulder is bent, the elbow is extended, and pre-shaping is performed. At this time, the user further extends the elbow and further bends the shoulder toward the grasping position. In addition, as the user approaches the virtual object to be grasped, the user makes the shape of the hand closer to the shape of the virtual object. The appearance of the pre-shaping movement is detected on the basis of a preparation movement (hand shape) of a body part to perform the grasping movement, or the like, acquired by an electromyograph or motion capture.

At the timing of the pre-shaping stage, the presentation control unit 19 changes the shape, size, contour, and the like of the virtual object to be grasped.

In the stage of the grasping movement, the hand is moved to the target. At this time, the user makes the hand reach the virtual object to be grasped. The appearance of the grasping movement is detected on the basis of the number of hands reaching the virtual object, the direction of moving the hands, the number of fingers to be used, the angle of the fingers, and the like acquired by motion capture.

At the timing of the grasping movement stage, the presentation control unit 19 performs feedback different from the aspect of the virtual object predicted by the user.

Note that, although the example in which the grasping movement is detected as the interaction has been described above, the interaction is not limited to the grasping movement, and various user operations can be detected as the interaction. In this case, the interaction is divided into, for example, five stages of recognition, preliminary movement, reaching movement, immediately preceding preparation movement, and main movement corresponding to five stages of recognition, bending, reaching movement, pre-shaping, and grasping movement. The number of interaction stages is not limited to five, and the interaction can be divided into any number of stages.

Returning to Fig. 1, the presentation control unit 19 selects a stimulus suitable for the stage of interaction as described above from among stimuli registered in the immersive feeling reduction stimulation DB 20 or newly generates a stimulus to present to the user. The presentation control unit 19 intervenes in the interaction by presenting a stimulus. The timing of presenting the stimulus is determined on the basis of the intervention type information. Specifically, a stimulus is presented at a timing corresponding to the immersion degree estimated by the immersion degree estimation unit 18 and the target value of the immersion degree indicated by the intervention type information. For example, at the timing when the immersion degree is equal to or greater than the target value and at the timing of a scene in which a virtual object capable of changing the visual characteristic is presented to the user, the visual characteristic of the virtual object changes.

In the immersive feeling reduction stimulation DB 20, each stage of interaction and a stimulus suitable for the stage are registered in association with each other.

The presentation unit 21 includes a head-mounted display, a display, a speaker, and the like. The presentation unit 21 presents content or presents a stimulus for reducing an immersive feeling for each interaction stage under the control of the presentation control unit 19.

### <2. Operation of Information Processing System>

In the information processing system 1, for example, a target value of an immersion degree is set according to purposes of the following (A) to (C), and presentation of a stimulus for reducing the immersion degree is performed at a timing based on the target value.

### (A) A case where it is not desired to immerse the user from the beginning

Examples of the case where it is not desired to immerse the user in the content from the beginning include a case where it is difficult to distinguish between virtual reality and reality if the user is immersed too much, and there is a risk of imposing a burden on the mind and body, and a case where there is a concern that visual information or a stimulus given to the brain may adversely affect the body.

For example, when experiencing content with a high psychological burden, such as VR guillotine, there is a possibility of receiving a very strong mental impact. Furthermore, in a case where the avatar operated by the user himself/herself is damaged unviable by a person or causes an illusion that the user himself/herself dies in the content, there is a possibility that the user's brain recognizes the information as realistic information.

In a case where it is not desired to immerse the content from the beginning, the information processing system 1 presents a stimulus so that the immersion degree does not approach 100% from the beginning of the content, for example.

### (B) A case where it is desired to maintain an appropriate immersive state

Examples of an example in which it is desired to maintain an appropriate immersive state include communication performed in a virtual space.

In order to enjoy and facilitate communication, presentation for presenting an avatar, an effect, or the like is performed. However, if the user gets used to the state, there is a possibility that a gap is felt when the user returns to reality, and communication in reality is hindered. Furthermore, there is a possibility that the user continues the communication without being able to notice the exhaustion of the communication partner or the change in mood due to the presentation by the avatar or the like. In the virtual space, ideal communication and communication at will can be performed, and thus, there is a possibility that the user feels that the virtual reality is real and escapes from the reality.

In a case where it is desired to maintain an appropriate immersive state, for example, the information processing system 1 presents a stimulus such that a state where the immersion degree is 100% is maintained as much as possible and the immersion degree does not exceed 100%.

### (C) Desired to return from immersive state to reality (desired to be in non-immersive state)

As an example in which it is desired to return from the immersive state to the reality, there is an example in which an action in the virtual space reaches the real state due to the excessive immersive state continuing for a long time.

For example, the user may be injured or injure someone by hitting his/her body against a surrounding wall or a person because the user is too immersed in the game while wearing the head-mounted display. In addition, there is a possibility that the user falls into a broad sense of mind control by arbitrary guidance of a content provider, such as wasting time and money while staying in a virtual space in which VR shopping is possible or a space of a billing game system for a long time.

In a case where it is desired to return from the immersive state to the reality, the information processing system 1 presents a stimulus to guide the immersion degree from 100% to less than 50%, for example.

Hereinafter, the operation of the information processing system 1 according to each purpose of the above (A) to (C) will be described.

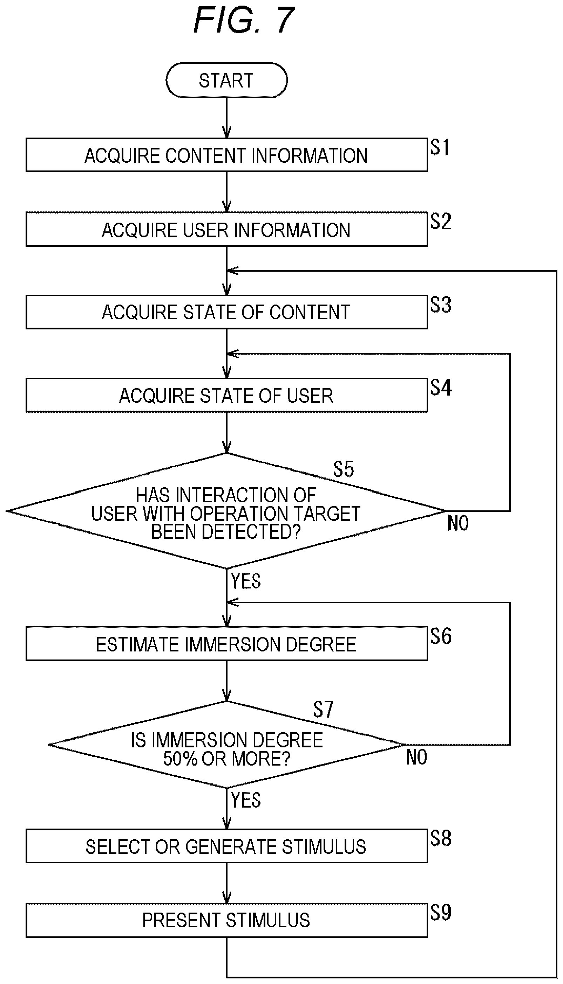

Processing performed by the information processing system 1 in a case where immersion is not desired from the beginning (purpose of (A)) will be described with reference to the flowchart of Fig. 7. The processing of Fig. 7 is started, for example, when presentation of content by the presentation unit 21 is started.

In step S1, the content state acquisition unit 15 acquires content information from the content DB 16.

In step S2, the user state acquisition unit 12 acquires user information from the user DB 13. For example, the user state acquisition unit 12 acquires, as the user information, the attribute of the user, the information of the user related to vision, and the information indicating the play history of the past games of the user.

In step S3, the content state acquisition unit 15 acquires the state of the content on the basis of the sensor data of the content sensor 14. For example, the content state acquisition unit 15 acquires the scene and the viewing state of the content presented to the user as the state of the content.

In step S4, the user state acquisition unit 12 acquires the state of the user on the basis of the sensor data of the user sensor 11. For example, the user state acquisition unit 12 acquires, as the state of the user, a line-of-sight fixed position and a gaze time of the user by eye tracking, or an utterance frequency of the user by a microphone. Furthermore, the user state acquisition unit 12 acquires, as the state of the user, a time history such as a viewing time, an operation start time, an operation time, and a response time of the content of the user, or acquires a peripheral device or a connected device such as a head-mounted display or a data glove worn by the user or a dome-type display used by the user to view the content.

The interaction detection unit 17 detects the interaction of the user with the operation target on the basis of the state of the user acquired by the user state acquisition unit 12. For example, the interaction detection unit 17 determines whether a virtual object as an operation target is recognized or gazed on the basis of the line-of-sight stay time. In a case where a plurality of virtual objects is simultaneously presented to the user, the interaction detection unit 17 detects which virtual object the user has paid attention to and where on the virtual object the user's attention is being directed. In step S5, the interaction detection unit 17 determines whether or not an interaction has been detected.

In a case where it is determined in step S5 that the interaction has not been detected, the processing returns to step S4, and acquisition of the state of the user is repeated.

On the other hand, in a case where it is determined in step S5 that the interaction has been detected, in step S6, the immersion degree estimation unit 18 estimates the immersion degree on the basis of the interaction detected by the interaction detection unit 17.

In step S7, the presentation control unit 19 determines whether or not the immersion degree is 50% or more set as the target value.

In a case where it is determined in step S7 that the immersion degree is less than 50%, the processing returns to step S6, and the estimation of the immersion degree is repeated.

On the other hand, in a case where it is determined in step S7 that the immersion degree is 50% or more, the processing proceeds to step S8. For example, in a case where the line-of-sight stays or follows the region where the virtual object that is likely to attract negative attention is presented for 3000 ms or more, the immersion degree estimation unit 18 estimates that the immersion degree is 50%, and the presentation control unit 19 starts presentation of a stimulus for reducing the immersion degree. Note that the accuracy of estimating the immersion degree and the like may be changed on the basis of the relationship between the characteristics of the virtual object and the interaction.

In step S8, the presentation control unit 19 selects or newly generates a stimulus suitable for the interaction stage from among the stimuli registered in the immersive feeling reduction stimulation DB 20. For example, the presentation control unit 19 causes the presentation unit 21 to present a stimulus that moves the position of the virtual object or changes the size or shape of the virtual object. Specifically, the presentation control unit 19 presents a stimulus for controlling the visual characteristics to be processed on the dorsal path in the user's brain, such as making the stereoscopic representation of the virtual object flat or changing the motion of the virtual object from smooth motion to jerky motion.

Note that, in order to present a stimulus around an operation target to which the user is paying attention, the presentation control unit 19 selects a stimulus presented in the vicinity of a region where a line-of-sight is detected or a stimulus presented in a conspicuous place in a scene presented to the user, and performs visual presentation that makes the user feel strangeness with respect to the operation target itself. Since it is necessary to present the stimulus in an easy-to-understand manner to some extent, the change of the virtual object is large to some extent and presented in an easy-to-understand manner.

In step S9, the presentation unit 21 presents a stimulus suitable for the stage of interaction to the user under the control of the presentation control unit 19.

Thereafter, the processing returns to step S3, and the subsequent processing is repeatedly performed.

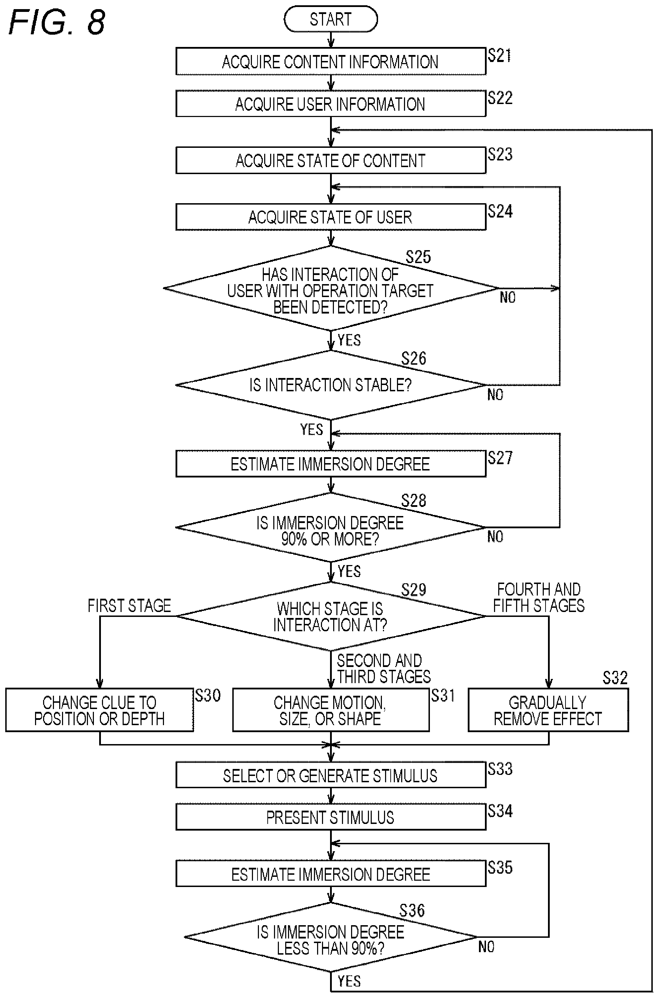

Next, processing performed by the information processing system 1 in a case where it is desired to maintain an appropriate immersive state (the purpose of (B)) will be described with reference to the flowchart of Fig. 8. The processing of Fig. 8 is started, for example, when the presentation unit 21 starts presentation of content for providing communication with an avatar of another user.

In step S21, the content state acquisition unit 15 acquires content information from the content DB 16.

In step S22, the user state acquisition unit 12 acquires user information from the user DB 13. For example, the user state acquisition unit 12 acquires user attributes and user information related to vision as user information.

In step S23, the content state acquisition unit 15 acquires the state of the content on the basis of the sensor data of the content sensor 14. For example, the content state acquisition unit 15 acquires the scene and the viewing state of the content presented to the user as the state of the content. Furthermore, the content state acquisition unit 15 also acquires a state such as an utterance frequency and an utterance tone of another user who is a communication partner, as the state of the content.

In step S24, the user state acquisition unit 12 acquires the state of the user on the basis of the sensor data of the user sensor 11. For example, the user state acquisition unit 12 acquires, as the state of the user, a line-of-sight fixed position and a gaze time of the user by eye tracking, and acquires an utterance frequency and an utterance tone of the user by a microphone. Furthermore, the user state acquisition unit 12 acquires a time history such as a viewing time, an operation start time, an operation time, and a response time of the content of the user as the state of the user.

The interaction detection unit 17 detects the interaction of the user with the operation target on the basis of the state of the user acquired by the user state acquisition unit 12. For example, the interaction detection unit 17 determines whether an avatar of another user is recognized or attention is paid to an avatar of another user on the basis of the user's line-of-sight stay time and the tracking of the line-of-sight. In a case where communication with a plurality of avatars is performed, the interaction detection unit 17 detects which avatar the user is paying attention to and what part of the avatar the user is paying attention to. Note that this interaction is detected in order to detect the stable state of the interaction in step S26. In step S25, the interaction detection unit 17 determines whether or not an interaction has been detected.

In a case where it is determined in step S25 that the interaction has been detected, in step S26, the interaction detection unit 17 determines whether or not the interaction is stable. For example, in a case where the interaction continues for a time defined in advance for each content, the interaction detection unit 17 determines that the interaction is stable.

In a case where it is determined in step S26 that the interaction is not stable or in a case where it is determined in step S25 that the interaction has not been detected, the processing returns to step S24, and acquisition of the state of the user is repeated.

On the other hand, in a case where it is determined in step S26 that the interaction is stable, in step S27, the immersion degree estimation unit 18 estimates the immersion degree on the basis of the interaction detected by the interaction detection unit 17.

In step S28, the presentation control unit 19 determines whether or not the immersion degree is 90% or more set as the target value.

In a case where it is determined in step S28 that the immersion degree is less than 90%, the processing returns to step S27, and the estimation of the immersion degree is repeated.

On the other hand, in a case where it is determined in step S28 that the immersion degree is 90% or more, the interaction detection unit 17 detects the interaction of the user with the operation target. Note that this interaction is detected in order to select a stimulus suitable for the interaction stage in steps S29 to S32.

Thereafter, in step S29, the presentation control unit 19 determines which stage the interaction is at. Hereinafter, an example in which a grasping movement is detected as an interaction will be described. As described above, the stage of interaction is divided into five stages of recognition, bending, reaching movement, pre-shaping, and grasping movement, and hereinafter, each stage is also referred to as a first stage to a fifth stage.

In a case where it is determined in step S29 that the interaction is in the first stage (cognition), in step S30, the presentation control unit 19 determines to change at least the visual characteristic serving as a clue to the position or depth of the virtual object.

In a case where it is determined in step S29 that the interaction is in the second stage (bending) or the third stage (reaching movement), in step S31, the presentation control unit 19 determines to change at least one of the motion, the size, or the shape of the virtual object. For example, in a case where a content for performing communication with the virtual idol is presented to the user, when the user reaches out to the avatar of the virtual idol, the presentation control unit 19 presents a stimulus that the avatar of the virtual idol runs away or becomes planar.

In a case where it is determined in step S29 that the interaction is in the fourth stage (pre-shaping) or the fifth stage (grasping movement), in step S32, the presentation control unit 19 determines to gradually remove at least the effect of the virtual object. For example, when the user tries to hold the hand of the virtual idol avatar, a stimulus is presented so that the user remembers that the virtual idol avatar is not authentic, such as the size and shape of a part or all of the virtual idol avatar changes. Furthermore, in a case where content that performs presentation in which an avatar is superimposed on another user who actually exists around the user is presented to the user, the presentation control unit 19 presents a stimulus that makes the user reminiscent of reality by occasionally canceling presentation so as not to partially present an effect to the avatar of the other user or the other user.

After the processing in any one of steps S30 to S32 is performed, in step S33, the presentation control unit 19 selects or newly generates the stimulus determined as described above from among the stimuli registered in the immersive feeling reduction stimulation DB 20.

Note that the presentation control unit 19 presents a stimulus to be presented in the vicinity of the region where the line-of-sight is detected in order to present a stimulus around the operation target to which the user is paying attention. For example, the presentation control unit 19 presents a stimulus that changes a part of an avatar itself of another user as an operation target or a visual characteristic of a virtual object presented around the avatar (another user).

In step S34, the presentation unit 21 presents a stimulus suitable for the stage of interaction to the user under the control of the presentation control unit 19.

In step S35, the immersion degree estimation unit 18 estimates the immersion degree again.

In step S36, the presentation control unit 19 determines whether or not the immersion degree is less than 90%.

In a case where it is determined in step S36 that the immersion degree is 90% or more, the processing returns to step S35, and the estimation of the immersion degree is repeated in a state where the stimulus for reducing the immersion degree is continuously presented until the immersion degree becomes less than 90%.

In a case where it is determined in step S36 that the immersion degree is less than 90%, the processing returns to step S23, and the subsequent processing is repeatedly performed.

Note that, in the processing of Fig. 8, the processing of steps S25 and S26 may be skipped, and the estimation of the immersion degree and the presentation of the stimulus may be performed regardless of the stable state of the interaction.

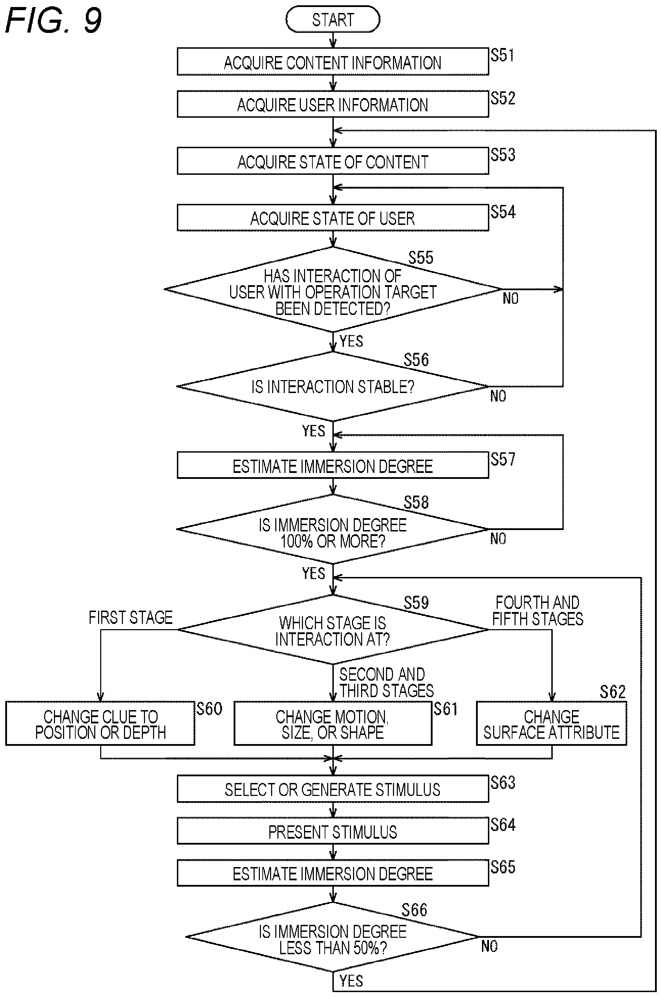

Next, with reference to the flowchart in Fig. 9, processing performed by the information processing system 1 in a case where it is desired to return from the immersive state to reality (the purpose of (C)) will be described. The processing of Fig. 9 is started, for example, when the presentation of the content is started in a situation where there is a possibility that an accident occurs due to an action in the virtual space actually reaching the user because the user is too enchanted.

In step S51, the content state acquisition unit 15 acquires content information from the content DB 16.

In step S52, the user state acquisition unit 12 acquires user information from the user DB 13. For example, the user state acquisition unit 12 acquires user attributes and user information related to vision as user information.

In step S53, the content state acquisition unit 15 acquires the state of the content on the basis of the sensor data of the content sensor 14. For example, the content state acquisition unit 15 acquires the scene and the viewing state of the content presented to the user as the state of the content.

In step S54, the user state acquisition unit 12 acquires the state of the user on the basis of the sensor data of the user sensor 11. For example, the user state acquisition unit 12 acquires, as the state of the user, a line-of-sight fixed position and a gaze time of the user by eye tracking, and acquires an utterance frequency and an utterance tone of the user by a microphone. Furthermore, the user state acquisition unit 12 acquires a time history such as a viewing time, an operation start time, an operation time, and a response time of the content of the user as the state of the user.

The interaction detection unit 17 detects the interaction of the user with the operation target on the basis of the state of the user acquired by the user state acquisition unit 12. For example, the interaction detection unit 17 determines whether the user recognizes the virtual object or pays attention to the virtual object on the basis of the line-of-sight stay time and the tracking of the line-of-sight of the user. In a case where a plurality of virtual objects is simultaneously presented to the user, the interaction detection unit 17 detects which virtual object the user has paid attention to and where in the virtual object the user has paid attention to. Note that this interaction is detected in order to detect the stable state of the interaction in step S56. In step S55, it is determined whether or not an interaction has been detected.

In a case where it is determined in step S55 that the interaction has been detected, in step S56, the interaction detection unit 17 determines whether or not the interaction is stable. For example, in a case where the interaction continues for a time defined in advance for each content, the interaction detection unit 17 determines that the interaction is stable.

In a case where it is determined in step S56 that the interaction is not stable or in a case where it is determined in step S55 that the interaction has not been detected, the processing returns to step S54, and acquisition of the state of the user is repeated.

On the other hand, in a case where it is determined in step S56 that the interaction is stable, in step S57, the immersion degree estimation unit 18 estimates the immersion degree on the basis of the interaction detected by the interaction detection unit 17.

For example, the immersion degree estimation unit 18 estimates the immersion degree so that the value increases in the order of the following cases (a) to (d).
(a) A case where the following (b) to (d) do not apply
(b) A case where the line-of-sight stays or follows a virtual object that is likely to attract attention regardless of whether the virtual object is positive or negative for 3000 ms or more
(c) A case where the interaction of the second stage or the third stage is recognized more than once
(d) A case where the interaction of the fourth stage or the fifth stage is recognized more than once

In step S58, the presentation control unit 19 determines whether or not the immersion degree is 100% or more set as the first target value.

In a case where it is determined in step S58 that the immersion degree is less than 100%, the processing returns to step S57, and the subsequent processing is performed.

On the other hand, in a case where it is determined in step S58 that the immersion degree is 100% or more, the interaction detection unit 17 detects the interaction of the user with the operation target. Note that this interaction is detected in order to select a stimulus suitable for the interaction stage in steps S59 to S62.

Thereafter, in step S59, the presentation control unit 19 determines which stage the interaction is at. Hereinafter, an example in which a grasping movement is detected as an interaction will be described.

In a case where it is determined in step S59 that the interaction is in the first stage, in step S60, the presentation control unit 19 determines to change at least the visual characteristic serving as a clue to the position or depth of the virtual object.

In a case where it is determined in step S59 that the interaction is in the second stage or the third stage, in step S61, the presentation control unit 19 determines to change at least one of the motion, the size, or the shape of the virtual object. For example, when the user extends a hand to the virtual object, the presentation control unit 19 presents a stimulus that causes the virtual object to escape or become planar.

In a case where it is determined in step S59 that the interaction is in the fourth stage or the fifth stage, in step S62, the presentation control unit 19 determines to change at least the surface attribute of the virtual object. For example, when the user tries to hold the virtual object, the presentation control unit 19 presents a stimulus that changes the visual characteristic of the virtual object processed on the reflective dorsal path according to the stage of interaction, such as changing the size and shape of a part or the whole of the virtual object.

After the processing in any one of steps S60 to S62 is performed, in step S63, the presentation control unit 19 selects or newly generates the stimulus determined as described above from among the stimuli registered in the immersive feeling reduction stimulation DB 20.

In step S64, the presentation unit 21 presents a stimulus suitable for the stage of interaction to the user under the control of the presentation control unit 19.

In step S65, the immersion degree estimation unit 18 estimates the immersion degree again.

In step S66, the presentation control unit 19 determines whether or not the immersion degree is less than 50% that is the second target value.

In a case where it is determined in step S66 that the immersion degree is 50% or more, the processing returns to step S59, and the presentation unit 21 continues to present a stimulus for reducing the immersion degree until the immersion degree becomes less than 50%. Note that, in order to gradually bring the user back to reality from the immersive state, the presentation unit 21 continues to present a stimulus with a feeling of strangeness that reduces the authenticity of the virtual object in accordance with the stage of interaction.

In a case where it is determined in step S66 that the immersion degree is less than 50%, the processing returns to step S53, and the subsequent processing is repeatedly performed.

As described above, in the information processing system 1, the stimulus for changing the virtual object according to the stage of the interaction of the user with the virtual object is presented to the user at the timing according to the immersion degree of the user in the content.

By changing the visual characteristics of the virtual object as processed in the dorsal path of the user's brain at the timing of each interaction stage, it is possible to reduce the immersion degree in the content without applying a psychological burden to the user.

Changing the visual characteristics of the virtual object at the timing of the precursor stage, including the cognitive stage and the preliminary movement stage, can work more unconsciously. In a case where the immersion degree is equal to or greater than the predetermined threshold value, the information processing system 1 changes the visual characteristic of the virtual object at the timing of the precursor stage, so that the user can recognize that the virtual object is a virtual thing in a form that is not so conscious. This makes it possible to prevent the immersion degree from rapidly decreasing due to the user being made to recognize that the virtual object is a virtual thing while the user is immersed in the content.

In a case where the immersion degree is lower than a predetermined threshold value, it is not necessary to consider that the immersion degree rapidly decreases. Therefore, the information processing system 1 can change the visual characteristic of the virtual object at the timing of the precursor stage and the timing of the main stage including the stage of the reaching movement, the stage of the pre-behavior movement, and the stage of the behavior movement. Furthermore, in a case where the immersion degree is lower than a predetermined threshold value, the information processing system 1 can change the virtual object at a timing before the interaction occurs.

Furthermore, in the information processing system 1, the immersion degree of the user is estimated on the basis of the content of the interaction. Since the state of the user can be accurately estimated on the basis of the content of the interaction, the immersion degree of the user can also be accurately estimated. Furthermore, the information processing system 1 can also evaluate the interaction and the stability of the content itself on the basis of the content of the interaction.

### <3. Use Case>

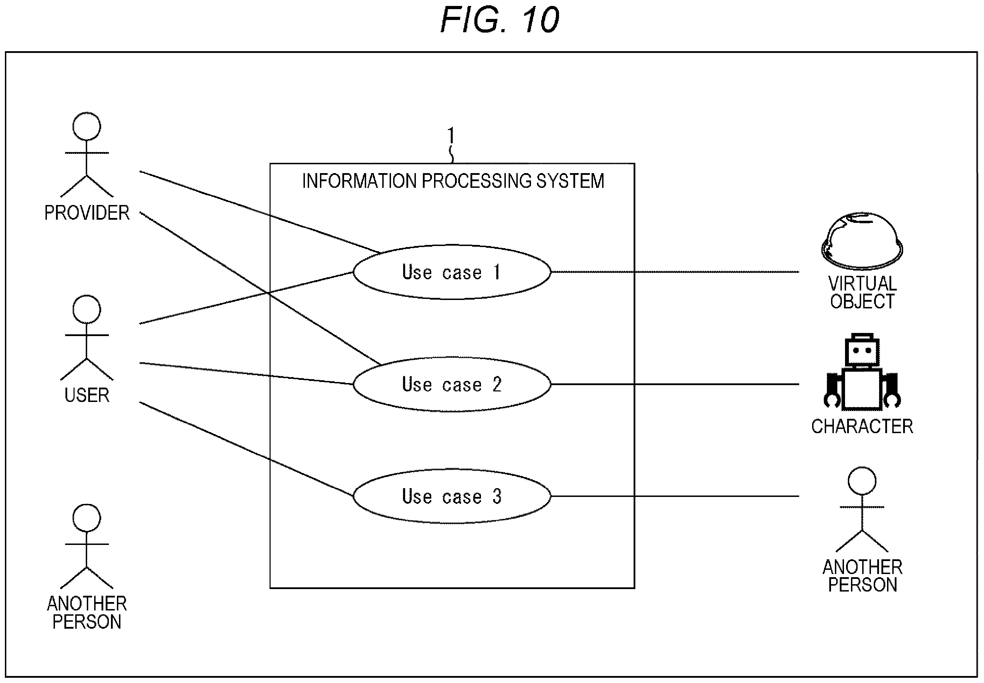

Fig. 10 is a diagram illustrating a use case of the information processing system 1.

As illustrated on the left side of Fig. 10, as a stakeholder that uses the information processing system 1, there are conceivable three parties including a provider and a user of the information processing system 1, and another person (another user) who uses the information processing system 1 together with the user.

In the use case 1, as illustrated on the right side of Fig. 10, a virtual object is exemplified as a target with which the stakeholder forms an interaction via the information processing system 1. In the use case 2, a character imitating a person is exemplified as a target with which the stakeholder forms interaction via the information processing system 1. In the use case 3, another person is exemplified as a target with which the stakeholder forms an interaction via the information processing system 1.

Note that the virtual object, the avatar of the character, and the avatar of the other person, which are targets of the interaction, are all virtual things in the virtual space.

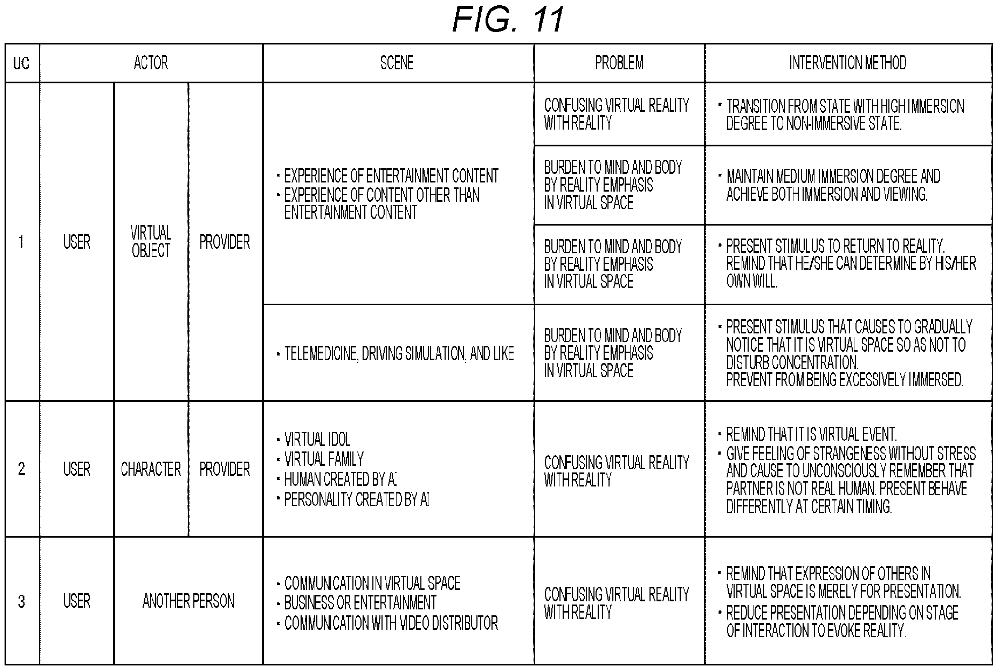

Fig. 11 is a diagram illustrating an example of problems in each use case (UC) and an intervention method of the information processing system 1.

As illustrated in Fig. 11, in the use case 1, the actors in content are a user, a virtual object, and a provider.

As a scene of the use case 1, there are scenes such as an experience of an entertainment content and an experience of content other than the entertainment content. In such a scene, as a first problem, there is a problem that virtual reality and reality are confused.

For example, in a case of experiencing content using a head-mounted display or a dome-type display, a user may think that they are moving in a virtual space, but may actually wave their hands around or the like and end up hitting a real-world wall and injuring themselves. When the user feels that the virtual object is a part of his/her hand like the rubber hand illusion, the user may feel that the hand hits the object in a situation where the hand does not actually hit the object, and the user may vigorously retreat his/her hand and hit something. In addition, illusion of the virtual object as a part of the user's own body causes the user to have a feeling of body extension or the like, which leads to an accident in reality such as an accident in which the user stretches his/her foot to an object and steps down although the user cannot actually reach the object.

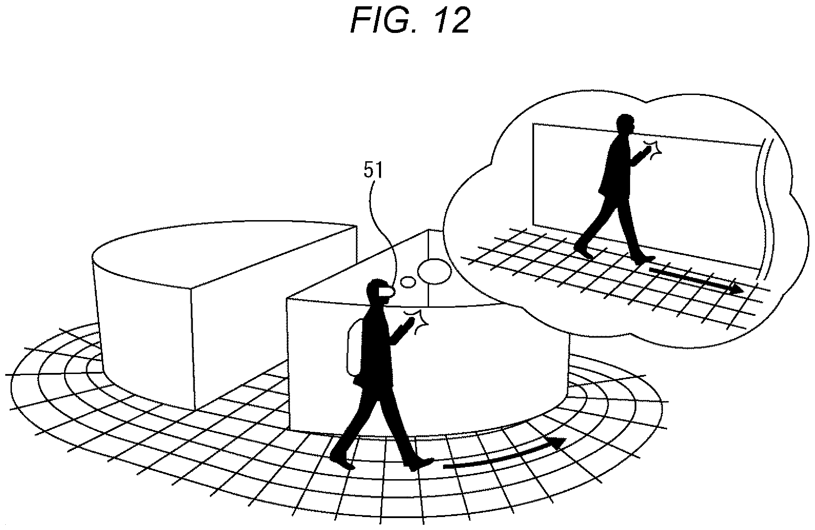

As illustrated in Fig. 12, by presenting content that makes the user wearing the head-mounted display 51 misunderstand that the user is walking on a straight road in the virtual space although the user actually walks along the elliptical wall, the user may continue walking infinitely.

As described above, there is a possibility that the user is too immersed in a game or the like with high reality, and the action in virtual reality reaches the reality.

Therefore, the information processing system 1 presents, to the user, a stimulus for transitioning from a state with a high immersion degree to a non-immersive state for the purpose of returning from the immersive state to reality (the purpose of (C)). For example, the information processing system 1 presents a stimulus for guiding an immersion degree from 100% to 80% or less to the user.

Specifically, the information processing system 1 senses a motion of a hand or the like with a data glove in a case where the user uses the head-mounted display, and senses a motion of a hand or the like with a marker or the like in a case where the user uses the dome-type display. In a case where the interaction after the second stage is detected several times, the information processing system 1 presents a stimulus suitable for the stage of the interaction. For example, when the user tries to extend a hand toward the virtual object, the user feels strangeness by deforming a part of the virtual object, causing the virtual object to move unpredictably, or the like.

Note that the visual characteristics of all virtual objects included in the content are not made changeable, but rather virtual objects whose visual characteristics can be changed are presented in a scene in which the presentation of a stimulus is assumed in advance. The virtual object capable of changing the visual characteristic is presented at a position where the user's attention is naturally attracted or a position where the user is likely to stay.

Presenting a stimulus to reduce the immersion degree in a scene that is a highlight of the content may reduce the enjoyment, so the information processing system 1 presents a stimulus in a scene that does not reduce the enjoyment on the basis of the intervention type information indicating whether or not intervention is possible for each scene registered in the content DB 16.

In a case where the immersion degree is predicted to increase on the basis of the user information and the content information, the information processing system 1 starts presentation of a stimulus at the precursor stage of the interaction to reduce the psychological burden on the user.

Note that the information processing system 1 can also generate an event that causes the user's interaction with the virtual object on the basis of the difference between the immersion degree and the target value. For example, in a case where the interaction has not occurred but it is desired to reduce the immersion degree, the information processing system 1 can present a stimulus according to the stage of the interaction that has occurred according to the event.

Returning to Fig. 11, in the scene of the use case 1 such as the experience of the entertainment content and the experience of the content other than the entertainment content, as the second problem, there is a problem that a burden is applied to the mind and body by the reality emphasis in the virtual space.

For example, when experiencing content with a high psychological burden, such as VR guillotine, there is a possibility of receiving a very strong mental impact. Furthermore, in a case where the avatar operated by the user himself/herself is damaged unviable by a person or causes an illusion that the user himself/herself dies in the content, there is a possibility that the user's brain recognizes the information as realistic information.

In this way, by spending a long time in the virtual space, integration of the user and the avatar becomes remarkable. The visual information and the stimulation given to the brain may adversely affect the body.

Therefore, for the purpose of preventing immersion from the beginning (the purpose of (A)), the information processing system 1 presents a stimulus so as to maintain a medium immersion degree and achieve both immersion and objective content viewing. For example, the information processing system 1 presents a stimulus so that the immersion degree does not approach 100% from the beginning of the content.

In this case, the information processing system 1 needs to allow the user to view the content while imparting objectivity as if the user views a video having realistic feeling on a television receiver without immersing the user so much that the user feels the video as a real experience from the start to the end of viewing the content. For this purpose, the information processing system 1 desirably presents a stimulus at a timing before interaction occurs. However, if a stimulus is presented even though the user is not about to perform any action on the virtual object, there is a concern that it may hinder the user from obtaining an immersive feeling or realistic feeling. Therefore, it is desirable to present the stimulus at the timing of the precursor stage of the interaction.

Furthermore, in a case where the interaction is detected, the information processing system 1 presents a stimulus that is likely to cause the user to feel strangeness as soon as the user sees it, such as changing the surface attributes of the virtual object to surface attributes that give the impression of characteristics that do not exist in reality (such as squishy glass) even when the interaction is in the precursor stage. In a case where the interaction at the main stage is detected, the information processing system 1 presents a stimulus that further makes the user feel strangeness.

Next, in the scene of the use case 1 such as the experience of the entertainment content and the experience of the content other than the entertainment content, as a third problem, there is a problem that a burden is applied to the mind and body by the reality emphasis in the virtual space different from the second problem.

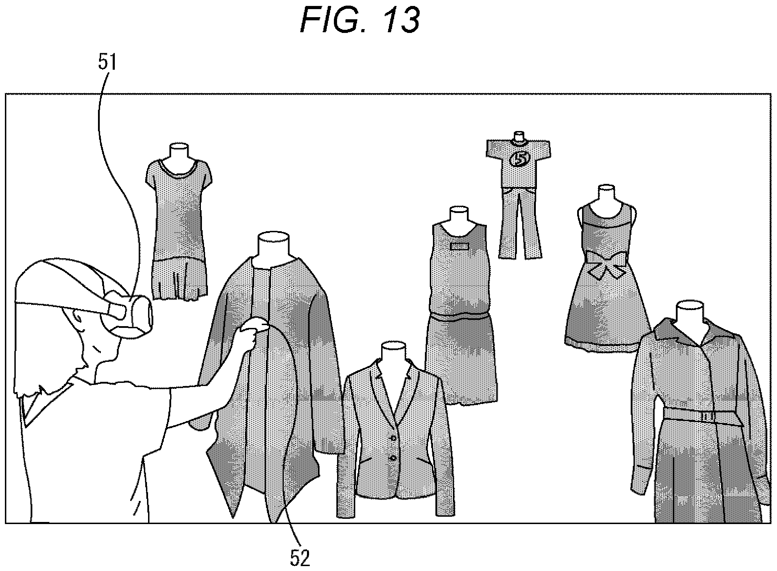

For example, as illustrated in Fig. 13, the user may utilize the head-mounted display 51 and the controller 52 to experience shopping in the virtual space as if he/she were looking at and purchasing clothing at a store. In a case where the user experiences a purchasing behavior, viewing of a video, or the like in the virtual space, if the user goes too far, the user may waste time and money, which may cause a psychological burden.

Even if the user intends to stop the experience soon, the user may continue the experience due to a provision on the provider side that makes it difficult to stop the experience, such as an advertisement function or recommendation by artificial intelligence (AI). As described above, there is a possibility that the user falls into a broad sense of mind control by arbitrary guidance of the content provider.

As described above, since it becomes a problem that the user continues to stay in the virtual space in which the user experiences the content, the information processing system 1 presents the user with a stimulus such as reminding the user of whether or not to continue the experience by the user's own will or forcibly prompting the user to exit from the virtual space in which the user experiences the content, for the purpose of returning from the immersive state to reality (the purpose of (C)). For example, the information processing system 1 presents a stimulus for guiding an immersion degree from 100% to 80% or less to the user.

Specifically, in a case where the immersion degree is 100% or more, the information processing system 1 presents the stimulus in the first stage of the interaction, and thereafter, presents the stimulus according to the stage of the interaction. In order to present a stimulus many times, the information processing system 1 needs to generate an event (situation) that the user intends to grasp a virtual object many times. The information processing system 1 generates an event such as selecting a product in shopping content or taking a virtual object in game content to occur until the immersion degree decreases.

Furthermore, in a case where the interaction after the second stage is detected several times, the information processing system 1 presents a stimulus in accordance with the stage of the detected interaction. For example, when the user tries to reach out to the virtual object, the information processing system 1 reminds that the experience can be interrupted by the user's will or whether or not to purchase the virtual object can be determined by presenting a stimulus such as moving the virtual object away from the user.

In a case where the immersion degree is 50% or less, the information processing system 1 can also present a stimulus that lowers the image quality of the entire content or a part of the content, regardless of the stage of interaction. In a case where the immersion degree is 50% or less, more types of visual characteristics may be changed in combination.

Referring back to Fig. 11, scenes such as telemedicine and driving simulation can be cited as scenes of use case 1. In such a scene, there is a problem that a burden is applied to the mind and body by the reality emphasis in the virtual space.

For example, in the virtual space, it is possible to create an environment in which it is easy to concentrate on work by blocking disturbance and emphasizing stimulation to be experienced. Therefore, as the work is continued, fatigue may accumulate unintentionally.

Since it becomes a problem that the user continues to stay in the virtual space in which the content is experienced, the information processing system 1 presents a stimulus that forcibly prompts the user to leave the virtual space in which the content is experienced, for the purpose of returning from the immersive state to the reality (the purpose of (C)). For example, the information processing system 1 presents a stimulus for guiding an immersion degree from 100% to 80% or less to the user.

Specifically, the information processing system 1 presents a stimulus that causes the user to gradually notice that the user is in the virtual space by deforming or shrinking the virtual object presented in the region of the visual field of the user so as not to disturb the concentration of the user. As a result, the information processing system 1 prevents the user from being excessively immersed.

Next, in the use case 2, the actors in content are a user, a character, and a provider. The character includes a virtual personality such as a virtual idol, a virtual family, a human created by AI, or a personality created by AI.

Examples of the scene of the use case 2 include a scene of communication with a virtual idol, a virtual family, a human created by AI, and a personality created by AI. In such a scene, there is a problem that virtual reality and reality are confused.

For example, by communicating with a non-existent character, the user may believe that the character exists, and may get a sense of loss or frustration when it is found that the character does not exist, and may not accept the reality. Furthermore, in a virtual space, it is possible to make the virtual personality of a deceased person speak or move as if the person were alive, which could cause problems.

In a case where two people communicate in a virtual space instead of meeting in real life to negotiate business deals or find a partner, there is a possibility that one person may allow a virtual personality to participate in the communication in place of themselves. In a case where this communication is successful, there is a possibility that the user will be disappointed when they actually meet due to the gap between their image of the other person and the reality.

In this way, in a case of communicating with a non-existent personality (such as a reproduction of a deceased person or a virtual idol), the user can experience the ideal communication or communication they want in the virtual space, which makes the user feel as if the virtual reality is real.

Therefore, the information processing system 1 presents a stimulus to remind the user that the experience is a virtual event for the purpose of maintaining an appropriate immersive state (the purpose of (B)). For example, the information processing system 1 presents a stimulus such that the immersion degree is maintained as low as 100% and the immersion degree does not exceed 100%.

Specifically, when the second-stage or third-stage interaction is detected several times, the information processing system 1 changes the visual characteristics of the avatar as a character so as to give a feeling of strangeness while finely simulating the shape and movement of a human, or causes the avatar to behave differently from reality. By presenting such a stimulus without stress (for example, without making the user feel creepy), the information processing system 1 causes the user to unconsciously remember that the communication partner is a virtual personality.

Furthermore, the information processing system 1 can detect, as the interaction of the user with the avatar, shaking hands with (contacting) the avatar, handing over an object to the avatar, or illustrating something to the avatar. In this case, the information processing system 1 presents a stimulus for reducing the immersion degree at the timing of these interactions. The timing of presenting the stimulus may be controlled by generating an event that causes interaction on the basis of the difference between the immersion degree and the target value. For example, in a case where the immersion degree is 100% or more, an event for handing over an object to an avatar is generated.

Since there is a concern that changing the visual characteristics of the avatar itself will increase the user's stress, it is preferable to change the visual characteristics of the virtual object presented around the avatar. The virtual object presented around the avatar can change the visual characteristics changed at the timing of the precursor stage. For example, at least one of the position or the depth of the virtual object can be changed.

When the interaction at the main stage is detected, it is conceivable to increase the frequency of presenting the stimulus for reducing the immersion degree. However, if the stimulus is frequently presented, there is a possibility of inhibiting the user from obtaining an immersive feeling. Therefore, it is preferable to present the stimulus at a frequency with which an appropriate immersive feeling can be obtained.

Note that the information processing system 1 can also select whether to present a stimulus for the purpose of not immersing the user from the beginning (the purpose of (A)) or to present a stimulus for the purpose of maintaining an appropriate immersive state (the purpose of (B)) according to the user's level of belief in virtual reality, which is specified on the basis of the user information.

Next, in the use case 3, the actors in the content are users and others.

Scenes of the use case 3 include scenes such as communication with others in a virtual space, business, entertainment, and communication with a video distributor in a virtual space. In such a scene, there is a problem that virtual reality and reality are confused.

For example, in the virtual space, since presentation in which an avatar, an effect, or the like is superimposed on another person is performed, even if the other person is actually exhausted, the user cannot accurately read the facial expression of the other person due to the presentation, and there is a possibility that the user continues the meeting or the like endlessly. Furthermore, as the user becomes accustomed to the presentation using the avatar of a smiling character or the effect of a smiling face, which is performed in order to facilitate communication, the user may feel a gap in the personality of another person when meeting in the real world, and the communication in the real world may be hindered.

Therefore, for the purpose of maintaining an appropriate immersive state (the purpose of (B)), the information processing system 1 presents a stimulus to remind the user that the expression of others in the virtual space is merely for presentation. For example, the information processing system 1 presents a stimulus such that the immersion degree is maintained as low as 100% and the immersion degree does not exceed 100%.

Specifically, the information processing system 1 does not present a stimulus at the timing immediately after the start of communication, and starts presentation of a stimulus at the timing after the communication is stabilized. Immediately after the start of communication, presentation for the purpose of smooth communication with others is performed. In order to remind the user that the expression of the other in the virtual space is merely an intervention, the information processing system 1 reduces the presentation by partially removing the effect superimposed on the body of the other person when the second-stage or third-stage interaction is detected several times. Furthermore, the information processing system 1 changes the position, depth, movement, shape, and the like of the virtual object presented around the avatar of the other person.

The information processing system 1 changes visual characteristics of a virtual thing such as an avatar of another person, an effect superimposed on another person, or a virtual object presented around an avatar of another person.

When the immersion degree is increased and the interaction at the main stage is detected, it is conceivable to increase the frequency of presentation of the stimulus. However, if a stimulus such as removal of an effect is frequently presented, there is a possibility that communication is disturbed. Therefore, it is preferable to present the stimulus at a frequency with which an appropriate immersive feeling can be obtained.

The information processing system 1 presents the stimulus little by little from the timing of the precursor stage, thereby unconsciously imprinting a feeling of strangeness, and imprinting that the interaction with another person is not a real event.

### <4. Modifications>

Changing a plurality of types of visual characteristics (for example, size and shape) in small increments may be more effective than changing one type of visual characteristic (for example, any of position, depth, movement, size, and shape) of a virtual object significantly or abruptly.

A plurality of combinations is prepared so that the degree of change in the visual characteristic and the frequency of changing the visual characteristic can be changed.

When a virtual object that is likely to attract attention is presented, detection of interaction and estimation of the immersion degree may be performed only on the basis of characteristics of the virtual object and characteristics of each scene, such as detecting interaction at a stage of recognition or estimating a high value as the immersion degree in a scene where the immersion degree is likely to be high.

### · Computer

The series of processing described above can be executed by hardware or by software. In a case where the series of processing is executed by software, a program included in the software is installed from a program recording medium on a computer incorporated in dedicated hardware, a general-purpose personal computer, or the like.

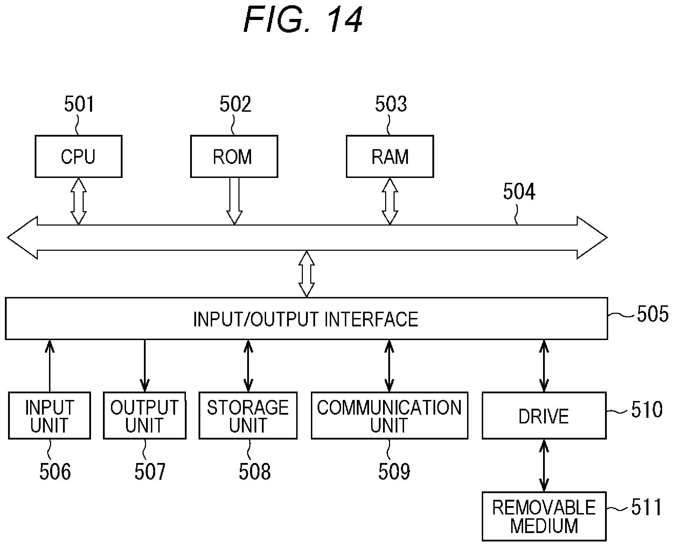

Fig. 14 is a block diagram illustrating a configuration example of hardware of a computer that executes the series of processing described above according to a program. A part of the configuration of the information processing system 1 includes, for example, a PC having a configuration similar to the configuration illustrated in Fig. 14.

A central processing unit (CPU) 501, a read only memory (ROM) 502, and a random access memory (RAM) 503 are mutually connected by a bus 504.

An input/output interface 505 is further connected to the bus 504. An input unit 506 including a keyboard, a mouse, and the like, and an output unit 507 including a display, a speaker, and the like are connected to the input/output interface 505. Furthermore, a storage unit 508 including a hard disk, a non-volatile memory, or the like, a communication unit 509 including a network interface or the like, and a drive 510 that drives a removable medium 511 are connected to the input/output interface 505.

In the computer configured as described above, for example, the CPU 501 loads a program stored in the storage unit 508 into the RAM 503 via the input/output interface 505 and the bus 504 and executes the program to execute the above-described series of processing.

For example, the program executed by the CPU 501 is recorded in the removable medium 511, or provided via a wired or wireless transmission medium such as a local area network, the Internet, or digital broadcasting, and then installed in the storage unit 508.

The program executed by the computer may be a program in which the processing is performed in time series in the order described in the present specification, or may be a program in which the processing is performed in parallel or at a necessary timing such as when a call is made.

Note that, in the present specification, a system means an assembly of a plurality of components (devices, modules (parts), and the like), and it does not matter whether or not all the components are located in the same housing. Therefore, a plurality of devices housed in separate housings and connected to each other via a network and one device in which a plurality of modules is housed in one housing are both systems.

Note that the effects described in the present specification are merely examples and are not limited, and other effects may be provided.

An embodiment of the present technology is not limited to the embodiment described above, and various modifications can be made without departing from the scope of the present technology.

For example, the present technology may be embodied in cloud computing in which a function is shared and executed by a plurality of devices via a network.

Further, each step described in the flowchart described above can be performed by one device or can be shared and performed by a plurality of devices.

Moreover, in a case where a plurality of pieces of processing is included in one step, the plurality of pieces of processing included in the one step can be executed by one device or executed by a plurality of devices in a shared manner.

### <Combination Example of Configuration>

The present technology can also have the following configurations.

(1) An information processing system including:
   a presentation control unit that presents content including an object to a user and changes the object according to a stage of interaction of the user with the object at a timing according to an immersion degree of the user with respect to the content.
(2) The information processing system according to (1),
   in which the presentation control unit changes a plurality of types of visual characteristics of the object.
(3) The information processing system according to (1) or (2),
   in which the presentation control unit changes a visual characteristic including at least one of a position, a depth, a motion, a size, or a shape of the object.
(4) The information processing system according to (3),
   in which the presentation control unit changes the object at a timing for each stage of the interaction.
(5) The information processing system according to (3) or (4),
   in which the stage of the interaction includes a recognition stage, a preliminary movement stage, a reaching movement stage, an immediately preceding preparation movement stage, and a main movement stage.
(6) The information processing system according to (5),
   in which the presentation control unit changes at least one of the position or the depth of the object at a timing of the recognition stage, changes at least one of the motion, the size, or the shape of the object at a timing of the preliminary movement stage or the reaching movement stage, changes at least one of the shape, the size, or a contour of the object at a timing of the immediately preceding preparation movement stage, and does not present a part of the object or changes a surface attribute of the object at a timing of the main movement stage.
(7) The information processing system according to (5) or (6),
   in which in a case where the immersion degree is equal to or greater than a predetermined threshold value, the presentation control unit changes the object at a timing of a precursor stage including the recognition stage and the preliminary movement stage.
(8) The information processing system according to any one of (5) to (7),
   in which in a case where the immersion degree is lower than a predetermined threshold value, the presentation control unit changes the object at a timing of a precursor stage including the recognition stage and the preliminary movement stage, and at a timing of a main stage including the reaching movement stage, the immediately preceding preparation movement stage, and the main movement stage.
(9) The information processing system according to (8),
   in which the presentation control unit changes the object at a timing before the interaction occurs in a case where the immersion degree is lower than a predetermined threshold value.
(10) The information processing system according to (1),
   in which the presentation control unit changes the object at a timing based on a target value of the immersion degree.
(11) The information processing system according to (10),
   in which the presentation control unit generates an event that causes the interaction to occur on the basis of a difference between the immersion degree and the target value.
(12) The information processing system according to (11),
   in which the content provides the user with communication with a partner predetermined, and
   the presentation control unit changes the object according to a stage of the interaction of the user with the partner for the communication.
(13) The information processing system according to (12),
   in which the presentation control unit changes at least one of an avatar of the partner for the communication or an effect on the partner for the communication as the object.
(14) The information processing system according to (12),
   in which the presentation control unit changes an avatar of the partner for the communication and the object presented around the partner for the communication other than an effect on the partner for the communication.
(15) The information processing system according to any one of (12) to (14),
   in which the partner for the communication is another user.
(16) The information processing system according to any one of (12) to (14),
   in which the partner for the communication is a virtual personality.
(17) The information processing system according to (16),
   in which the object presented around the partner for the communication is capable of changing at least one of a position or a depth.
(18) The information processing system according to any one of (12) to (17),
   in which the interaction includes that the user comes in contact with the partner for the communication or that the user hands over an object to the partner for the communication.
(19) The information processing system according to (18),
   in which the presentation control unit generates the event in which the user hands over an object to the partner for the communication on the basis of the difference between the immersion degree and the target value.
(20) An information processing method in which
   an information processing system is configured to:
   present content including an object to a user; and
   change the object according to a stage of interaction of the user with the object at a timing according to an immersion degree of the user with respect to the content.
(21) The information processing system according to any one of (1) to (17),
   in which the interaction includes the user grasping the object.

### REFERENCE SIGNS LIST

- 1: Information processing system
- 11: User sensor
- 12: User state acquisition unit
- 13: User DB
- 14: Content sensor
- 15: Content state acquisition unit
- 16: Content DB
- 17: Interaction detection unit
- 18: Immersion degree estimation unit
- 19: Presentation control unit
- 20: Immersive feeling reduction stimulation DB
- 21: Presentation unit

## Claims

1. An information processing system comprising:
a presentation control unit that presents content including an object to a user and changes the object according to a stage of interaction of the user with the object at a timing according to an immersion degree of the user with respect to the content.

2. The information processing system according to claim 1,
wherein the presentation control unit changes a plurality of types of visual characteristics of the object.

3. The information processing system according to claim 1,
wherein the presentation control unit changes a visual characteristic including at least one of a position, a depth, a motion, a size, or a shape of the object.

4. The information processing system according to claim 3,
wherein the presentation control unit changes the object at a timing for each stage of the interaction.

5. The information processing system according to claim 3,
wherein the stage of the interaction includes a recognition stage, a preliminary movement stage, a reaching movement stage, an immediately preceding preparation movement stage, and a main movement stage.

6. The information processing system according to claim 5,
wherein the presentation control unit changes at least one of the position or the depth of the object at a timing of the recognition stage, changes at least one of the motion, the size, or the shape of the object at a timing of the preliminary movement stage or the reaching movement stage, changes at least one of the shape, the size, or a contour of the object at a timing of the immediately preceding preparation movement stage, and does not present a part of the object or changes a surface attribute of the object at a timing of the main movement stage.

7. The information processing system according to claim 5,
wherein in a case where the immersion degree is equal to or greater than a predetermined threshold value, the presentation control unit changes the object at a timing of a precursor stage including the recognition stage and the preliminary movement stage.

8. The information processing system according to claim 5,
wherein in a case where the immersion degree is lower than a predetermined threshold value, the presentation control unit changes the object at a timing of a precursor stage including the recognition stage and the preliminary movement stage, and at a timing of a main stage including the reaching movement stage, the immediately preceding preparation movement stage, and the main movement stage.

9. The information processing system according to claim 8,
wherein the presentation control unit changes the object at a timing before the interaction occurs in a case where the immersion degree is lower than a predetermined threshold value.

10. The information processing system according to claim 1,
wherein the presentation control unit changes the object at a timing based on a target value of the immersion degree.

11. The information processing system according to claim 10,
wherein the presentation control unit generates an event that causes the interaction to occur on a basis of a difference between the immersion degree and the target value.

12. The information processing system according to claim 11,
wherein the content provides the user with communication with a partner predetermined, and
the presentation control unit changes the object according to a stage of the interaction of the user with the partner for the communication.

13. The information processing system according to claim 12,
wherein the presentation control unit changes at least one of an avatar of the partner for the communication or an effect on the partner for the communication as the object.

14. The information processing system according to claim 12,
wherein the presentation control unit changes an avatar of the partner for the communication and the object presented around the partner for the communication other than an effect on the partner for the communication.

15. The information processing system according to claim 12,
wherein the partner for the communication is another user.

16. The information processing system according to claim 12,
wherein the partner for the communication is a virtual personality.

17. The information processing system according to claim 16,
wherein the object presented around the partner for the communication is capable of changing at least one of a position or a depth.

18. The information processing system according to claim 12,
wherein the interaction includes that the user comes in contact with the partner for the communication or that the user hands over an object to the partner for the communication.

19. The information processing system according to claim 18,
wherein the presentation control unit generates the event in which the user hands over an object to the partner for the communication on a basis of the difference between the immersion degree and the target value.

20. An information processing method in which
an information processing system is configured to:
present content including an object to a user; and
change the object according to a stage of interaction of the user with the object at a timing according to an immersion degree of the user with respect to the content.
